# EUROPEAN PATENT APPLICATION

(11) **EP 4 491 704 A1**
(43) Date of publication of application: **15.01.2025**
(21) Application number: 24185837.2
(22) Date of filing: 01.07.2024
(51) Int. Cl.: C12N 1/00, C12N 1/20, B09C 1/10, C02F 3/34

(54) **SYNERGISTIC BIOREMEDIATION COMPOSITION**

(30) Priority: 30.06.2023 PT 2023118784
(71) Applicant: CIIMAR - Centro Interdisciplinar de Investigação Marinha e Ambiental, 4450-208 Matosinhos (PT); Universidade Do Porto, 4099-002 Porto (PT)
(72) Inventor: DE CAMPOS MUCHA, ANA PAULA, 4450-208 MATOSINHOS (PT); TOMASINO, MARIA PAOLA, 4450-208 MATOSINHOS (PT); RIBEIRO DE ALMEIDA, CRISTINA MARISA, 4450-208 MATOSINHOS (PT); PERDIGÃO MENDES, RAFAELA ALEXANDRA, 4450-208 MATOSINHOS (PT); DE VILAR CORREIA BRITO BÔTO, MARIA LUIS, 4450-208 MATOSINHOS (PT); PINTO MORA PINTO DE MAGALHÃES, CATARINA MARIA, 4450-208 MATOSINHOS (PT); MAGALHÃES CARVALHO, MARIA DE FÁTIMA, 4450-208 MATOSINHOS (PT); DA COSTA E SILVA RAMOS, SANDRA CRISTINA, 4450-208 MATOSINHOS (PT)
(74) Representative: Patentree

(57) **Abstract**

The present disclosure relates to a bioremediation synergistic composition for hydrocarbon pollutants degradation, comprising at least a bacterial strain of genus *Pseudomonas* or *Halopseudomonas,* preferably *Pseudomonas segetis* strain BDO24 or *Halopseudomonas* sp. strain BP4; at least a bacterial strain of genus *Alcanivorax* or *Alloalcanivorax,* preferably *Alcanivorax borkumensis* strain BP2 or *Alloalcanivorax* sp. strain BP14; and at least a bacterial strain of genus *Thalassospira,* preferably *Thalassospira mesophila* strain BP3.

## Description

### TECHNICAL FIELD

The present disclosure relates to a synergistic bioremediation composition for hydrocarbon pollutants degradation, comprising a synergistic mixture of: at least a bacterial strain of genus Pseudomonas or *Halopseudomonas* , preferably *Pseudomonas segetis* strain BDO24 or *Halopseudomonas* sp. strain BP4; at least a bacterial strain of genus *Alcanivorax or Alloalcanivorax* , preferably *Alcanivorax borkumensis* strain BP2 or *Alloalcanivorax sp.* strain BP14; and at least a bacterial strain of genus *Thalassospira,* preferably *Thalassospira mesophila* strain BP3.

*Alcanivorax borkumensis* strain BP2 was received on 6^{th} June 2023 at the Spanish Type Culture Collection (CECT) - International Depositary Authority under the Budapest Treaty, with the address C/ Catedrático Agustin Escardino, 9. 46980 Paterna (Valencia), Spain, under the accession number CECT 30863.

*Thalassospira mesophila* strain BP3 was received on 6^{th} June 2023 at the Spanish Type Culture Collection (CECT) - International Depositary Authority under the Budapest Treaty, with the address C/ Catedrático Agustin Escardino, 9. 46980 Paterna (Valencia), Spain, under the accession number CECT 30864.

*Halopseudomonas sp.* strain BP4 was received on 17^{th} May 2024 at the Spanish Type Culture Collection (CECT) - International Depositary Authority under the Budapest Treaty, with the address C/ Catedrático Agustin Escardino, 9. 46980 Paterna (Valencia), Spain, under the accession number CECT 31085.

*Alloalcanivorax sp.* strain BP14 was received on 17^{th} May 2024 at the Spanish Type Culture Collection (CECT) - International Depositary Authority under the Budapest Treaty, with the address C/ Catedrático Agustin Escardino, 9. 46980 Paterna (Valencia), Spain, under the accession number CECT 31086.

*Pseudomonas segetis* strain BDO24 was received on 6^{th} June 2023 at the Spanish Type Culture Collection (CECT) - International Depositary Authority under the Budapest Treaty, with the address C/ Catedrático Agustin Escardino, 9. 46980 Paterna (Valencia), Spain, under the accession number CECT 30862. *Pseudomonas segetis* strain BDO24 (CECT 30862) was previously identified as *Pseudomonas marincola,* nonetheless, it is currently identified as *Pseudomonas segetis* by the NCBI 16S rRNA database.

Recent changes in the taxonomy of the Pseudomonadaceae family have led to the delineation of three new genera, supplementary to *Pseudomonas,* which were previously included in the *Pseudomonas* genus - *Atopomonas, Halopseudomonas* and *Stutzerimonas* (Girard et al., 2023; Girard et al., 2021; Lalucat et al., 2022).

Also, some taxa previously grouped in the *Alcanivorax* genus, recently suffered a taxonomic update into 2 other genera: *Alloalcanivorax* and *Isoalcanivorax* (Rai et al., 2023).

### BACKGROUND

Hydrocarbon pollution resulting from anthropogenic activities threatens our marine ecosystems, whether by acute events of contamination, such as oil spills, or by chronic contamination. Mass scale oil spills have high media coverage and are extremely dangerous to the environment, pressuring governments and agencies to act fast to contain and tackle the spillage. Sometimes, a faster response might not be the most environmental-friendly or effective approach [1], as illustrated by the Deepwater Horizon oil spill in 2010, where chemical dispersants were applied in large scale to disperse the oil, harming the wildlife [2, 3]. Bioremediation technologies, on the other hand, have been considered promising ecologic alternatives to tackle oil spills, avoiding the negative implications as-sociated to physical-chemical techniques, like the introduction of chemical dispersants or burning the spilled oil [4-6].

Bioremediation can be divided into two strategies: biostimulation (BS), by adding nutrients or biosurfactants to the affected area to stimulate the oil-degrading metabolisms by the microbial community naturally present, and bioaugmentation (BA) where known oil-degrading microorganisms are added to increase their abundance among the local microbial community. A combination of these two methods has been proved to enhance hydrocarbon degradation [4, 7, 8]. Many groups of microorganisms have been reported to play a role on hydrocarbons degradation in the marine environment, such as the filamentous fungi genera *Aspergillus* and *Penicillium* [9] and the yeast genus *Candida* [10], nonetheless bacteria are considered the major intervenient in hydrocarbons biodegradation at sea [11]. Members of the classes Gammaproteobacteria, Alphaproteobacteria and Actinobacteria have been isolated from seawater and marine sediment samples showing hydrocarbon bioremediation potential [12-14]. These microorganisms have the advantages of not competing with the natural community for the carbon source, and do not cause the possible negative impact associated with the introduction of exogenous organisms [21, 22]. Recently, several laboratory studies highlighted the importance and potential of using native bacterial consortia to bioremediate petroleum hydrocarbons in impacted marine environments [5, 19, 20].

Even by doing effective laboratory experiments to test bioremediation agents or a combination of agents, the bioremediation efficiency on a real environment relies on various factors, such as, environmental conditions, the concentration and chemical composition of the oil spilled, the bioavailability of hydrocarbons, the concentration of nutrients present and the time taken to develop a tailor-made solution and effective bacterial consortium to apply after the oil spill [23-25].

Regarding application of bioremediation agents in open water system, such as seawater, the application of free cell bioremediation might be challenging as it might disperse in the water column [26]. To tackle this issue, some studies suggest the application of immobilized bacterial cells into carriers, biofilms or enzyme substances to enhance the hydrocarbons biodegradation performance [27-31]. Wang et al. [28] studied the application of immobilized bacteria in field tests (3 months), contaminated with crude oil and diesel and observed that the immobilized bacterial consortia performed better in the degradation of hydrocarbons. Hou et al. [31] observed an enhanced diesel bioremediation process by immobilising an oil-degrading bacteria *Acinetobacter sp.* to a novel carrier, when compared to an addition of free cells to the experiment. Despite the promising bioremediation results from these previous studies, most of the immobilized bacteria used in the process were not autochthonous for the site.

Field tests with the application of microbial agents in marine environment evidenced the effectiveness of bioaugmentation in the removal of oil from rocks and sediments at a beach impacted by the Nakhodka oil spill (1997) [32]. There are already a few microbial products in the market to tackle oil spills, besides nutrient fertilizers and other chemical compounds prepared for biostimulation.

A market study conducted by Villela et al. [33] on patents for microorganisms used in bioremediation revealed that the majority of them belong to microorganisms from the Bacteria group (368 out of 500 patents). Species from the genera *Pseudomonas, Rhodococcus, Acinetobacter,* and *Bacillus* rank among the top 10 bacterial agents for bioremediation. However, these patents are mostly focused on individual bacterial strains or consortia of exogenous microorganisms. Therefore, there is a need to identify and isolate autochthonous bacterial strains with high petroleum biodegradation potential to expand the range of native bacterial consortia products for hydrocarbon bioremediation. Studies on biomass production optimization, preparation of consortia for fied application and determine the necessary quantities for a real spill remediation.

These facts are disclosed in order to illustrate the technical problem addressed by the present disclosure.

### GENERAL DESCRIPTION

The present disclosure relates to a synergistic bioremediation composition for hydrocarbon pollutants degradation comprising a bacterial consortium.

The overall aim of the present invention is to provide an innovative, effective and environmentally friendly solution to tackle one of the most damaging sources of maritime pollution: spills of oil and maritime fuels. This solution is presented through the production of autochthonous microbial consortia with bioremediation capacity, supported by a cocktail of nutrients to stimulate microbial activity. By using autochthonous microorganisms to naturally degrade oil spills, this solution will avoid the introduction of additional chemical or biological additives that could disrupt the biological equilibrium of the affected ecosystem. The autochthonous bioremediation formulation is composed of different autochthonous microorganisms isolated along the Atlantic Iberian Peninsula Coast, most belonging to the genera *Pseudomonas, Halopseudomonas, Alcanivorax* , Alloalcanivorax and *Thalassospira.* When combined, surprisingly these microorganisms act synergistically and have the capability to degrade, in natural seawater, between 50% and 90% of petroleum hydrocarbons from different origins (e.g. crude oil, turbine oil, diesel oil). This invention also includes the workflow for the production of the microbial biomass necessary for the bioremediation formulation, by using environmentally friendly ingredients, namely byproducts from different industries, in line with the circular economy.

The present disclosure provides: optimization of the biomass production of three oil-degrading bacterial strains previously isolated from seawater ; (ii) test the viability and hydrocarbon degrading capacity of the isolated strains, individually or combined into a consortia; (iii) optimization of the ratio of the bacterial consortium biomass and oil for an efficient biodegradation of hydrocarbons and (iv) validation of the bioremediation efficiency of the optimized consortium in microcosms experiments with natural seawater.

The present disclosure represents a novelty in the market because, for the first time, it is proposed a bioremediation formulation reporting the usage of an autochthonous bacterial (i.e. microorganisms that are indigenous or native to a particular environment, and are totally adapted to survive and thrive in that environment) consortium as a bioremediation solution for marine environment contaminated by petroleum-derived products, namely along the Atlantic Iberian Coast, for petroleum hydrocarbons removal.

It was surprisingly found that a bioremediation composition comprising a synergistic mixture of at least a bacterial strain of genus *Pseudomonas or Halopseudomonas;* at least a bacterial strain of genus *Alcanivorax* or *Alloalcanivorax*; and at least a bacterial strain of genus *Thalassospira,* provides a synergistic effect by providing an enhanced degradation activity of petroleum hydrocarbons pollutants, namely diesel oil, crude oil and/or turbine oil. Preferably, said composition comprises a synergistic mixture at least *Pseudomonas segetis, Alcanivorax borkumensis* and *Thalassospira mesophile.*

Surprisingly, a bioremediation composition comprising a synergistic mixture of at least *Halopseudomonas* sp., *Alloalcanivorax* sp. and *Thalassospira mesophila* provides a synergistic effect by providing an enhanced degradation activity of petroleum hydrocarbons pollutants, namely diesel oil, crude oil and/or turbine oil.

An aspect of the present disclosure relates to a bioremediation composition for hydrocarbon pollutants degradation comprising a synergistic mixture of:
at least a bacterial strain of genus *Pseudomonas* or *Halopseudomonas;*
at least a bacterial strain of genus *Alcanivorax* or *Alloalcanivorax*; and
at least a bacterial strain of genus *Thalassospira;*
wherein the bacterial strain of genus *Pseudomonas or Halopseudomonas* is selected from a list consisting of: *Pseudomonas marincola, Pseudomonas oceani, Pseudomonas putida, Pseudomonas sabulinigri, Pseudomonas segetis, Pseudomonas sp. or Halopseudomonas sp., or combinations thereof;* preferably *Pseudomonas segetis or Halopseudomonas sp.;*
wherein the bacterial strain of genus *Alcanivorax* or *Alloalcanivorax* is selected from a list consisting of: *Alcanivorax borkumensis, Alcanivorax dieselolei, Alcanivorax marinus, Alcanivorax xenomutans, Alcanivorax* sp. or *Alloalcanivorax sp.,* or combinations thereof; preferably *Alcanivorax borkumensis or Alloalcanivorax sp.;*
wherein the bacterial strain of genus *Thalassospira* is selected from a list consisting of: *Thalassospira lucentensis, Thalassospira mesophila, Thalassospira xiamenensis* or *Thalassospira sp.,* or combinations thereof; preferably *Thalassospira mesophila.*

In a preferred embodiment, the *Pseudomonas marincola* strain is selected from a list consisting of: *Pseudomonas marincola* strain BPS, *Pseudomonas marincola* strain BP24, *Pseudomonas marincola* strain BP25, *Pseudomonas marincola* strain BP45, *Pseudomonas marincola* strain BTO19, *Pseudomonas marincola* strain BTO22; *the Halopseudomonas oceani* strain is selected from a list consisting of: *Halopseudomonas oceani* strain BTO18, *Halopseudomonas oceani* strain BTO20, *Halopseudomonas oceani* strain BTO37; the *Pseudomonas putida* strain is selected from a list consisting of *Pseudomonas putida* strain BDO8, *Pseudomonas putida* strain BDO20, *Pseudomonas putida* strain BDO31, *Pseudomonas putida* strain BDO49; the *Halopseudomonas sabulinigri* strain is selected from a list consisting of *Halopseudomonas sabulinigri* strain BTO13, *Halopseudomonas sabulinigri* strain BDO37; the *Pseudomonas segetis* strain is selected from a list consisting of *Pseudomonas segetis* strain BTO21, *Pseudomonas segetis* strain BTO44, *Pseudomonas segetis* strain BDO17, *Pseudomonas segetis* strain BDO22, *Pseudomonas segetis* strain BDO38, *Pseudomonas segetis* strain BDO46, *Pseudomonas segetis* strain BP43, *Pseudomonas segetis* strain BDO24; preferably *Pseudomonas segetis* strain BDO24; the *Pseudomonas* sp. or *Halopseudomonas* sp. strain is selected from a list consisting of *Halopseudomonas sp.* strain BP4, *Pseudomonas sp.* BP17, *Pseudomonas sp.* BDO6, *Pseudomonas sp.* BDO7, *Pseudomonas sp.* BDO11, *Pseudomonas sp. Pseudomonas sp.* BDO12, *Pseudomonas sp.* BDO26, *Pseudomonas sp.* BDO36, *Pseudomonas* sp. BDO40, *Pseudomonas sp.* BDO42, *Pseudomonas sp.* BDO43, *Pseudomonas sp.* BDO45, *Pseudomonas sp.* BDO52, *Pseudomonas sp.* BP35, *Pseudomonas sp.* BTO6, *Pseudomonas sp.* BTO17, preferably *Halopseudomonas* strain BP4.

In a preferred embodiment, the bacterial strain of genus *Pseudomonas* is *Pseudomonas segetis* strain BDO24.

In a preferred embodiment, the bacterial strain of genus *Halopseudomonas is Halopseudomonas sp.* strain BP4.

In an embodiment, the *Alcanivorax borkumensis* strain is selected from a list consisting of: *Alcanivorax borkumensis* strain BP2, *Alcanivorax borkumensis* strain BP7, *Alcanivorax borkumensis* strain BP37, *Alcanivorax borkumensis* strain BP41, *Alcanivorax borkumensis* strain BTO2, *Alcanivorax borkumensis* strain BTO28, *Alcanivorax borkumensis* strain BTO31, *Alcanivorax borkumensis* strain BTO32, *Alcanivorax borkumensis* strain BDO1, *Alcanivorax borkumensis* strain BDO5, *Alcanivorax borkumensis* strain BDO44; preferably *Alcanivorax borkumensis* strain BP2; the *Alloalcanivorax dieselolei* strain is selected from a list consisting of: *Alloalcanivorax dieselolei* strain BP18, *Alloalcanivorax dieselolei* strain BP33, *Alloalcanivorax dieselolei* strain BDO3, *Alloalcanivorax dieselolei* strain BDO14, *Alloalcanivorax dieselolei* strain BDO25, *Alloalcanivorax dieselolei* strain BDO28, *Alloalcanivorax dieselolei* strain BDO32, *Alloalcanivorax dieselolei* strain BDO33, *Alloalcanivorax dieselolei* strain BDO34, *Alloalcanivorax dieselolei* strain BDO41, *Alloalcanivorax dieselolei* strain BDO48; the *Alloalcanivorax marinus* strain is *Alloalcanivorax marinus* strain BDO29; the *Alcanivorax sp.* strain is selected from a list consisting of: *Alcanivorax* strain BP8, *Alcanivorax* strain BP40; the *Alloalcanivorax xenomutans* strain is *Alloalcanivorax xenomutans* strain BP21; and the *Alloalcanivorax sp.* strain is *Alloalcanivorax sp.* strain BP14.

In a preferred embodiment, the bacterial strain of genus *Alcanivorax* is *Alcanivorax borkumensis* strain BP2.

In a preferred embodiment, the bacterial strain of genus *Alloalcanivorax sp. is Alloalcanivoraxsp.* strain BP14.

In an embodiment, the *Thalassospira lucentensis* strain is selected from a list consisting of: *Thalassospira lucentensis* strain BP39, *Thalassospira lucentensis* strain BP15, *Thalassospira lucentensis* strain BDO19; the *Thalassospira mesophila* strain is selected from a list consisting of: *Thalassospira mesophila* strain BP3, *Thalassospira mesophila* strain BP30; preferably Thalassospira mesophila strain BP3; *the Thalassospira sp.* strain is selected from a list consisting of: *Thalassospira sp.* strain BDO15, *Thalassospira sp.* strain BDO23, *Thalassospira* sp.strain BTO3, *Thalassospira sp.* strain BTO45; and the *Thalassospira xiamenensis* strain is selected from a list consisting of: *Thalassospira xiamenensis* strain BDO13, *Thalassospira xiamenensis* strain BDO30.

In a preferred embodiment, the composition comprises 10⁶ - 10¹² CFU/L of a bacterial strain of genus *Pseudomonas;* preferably *Pseudomonas segetis* strain BDO24.

In a preferred embodiment, the composition comprises 10⁶ - 10¹² CFU/L of a bacterial strain of genus *Halopseudomonas;* preferably *Halopseudomonassp.* strain BP4.

In a preferred embodiment, the composition comprises 10⁶ - 10¹² CFU/L of a bacterial strain of genus *Alcanivorax;* preferably *Alcanivorax borkumensis* strain BP2.

In a preferred embodiment, the composition comprises 10⁶ - 10¹² CFU/L of a bacterial strain of genus *Alloalcanivorax*; preferably *Alloalcanivorax sp.* strain BP14.

In a preferred embodiment, the composition comprises 10⁶ - 10¹² CFU/L of a bacterial strain of genus *Thalassospira;* preferably *Thalassospira mesophila* strain BP3.

In a more preferred embodiment, the composition of the present disclosure comprises a mixture of *Pseudomonas segetis* strain BDO24, *Alcanivorax borkumensis* strain BP2 and *Thalassospira mesophila* strain BP3; preferably in a proportion 1:1:1 in CFU.

In a preferred embodiment, the composition of the present disclosure comprises a mixture of 10⁶ - 10¹² CFU/L of *Pseudomonas segetis* strain BDO24, 10⁶ - 10¹² CFU/L of *Alcanivorax borkumensis* strain BP2 and 10⁶ - 10¹² CFU/L of *Thalassospira mesophila* strain BP3.

In a preferred embodiment, the composition of the present disclosure comprises a mixture of 0.33×10⁹ CFU/L of *Pseudomonas segetis* strain BDO24, 0.33×10⁹ CFU/L of *Alcanivorax borkumensis* strain BP2 and 0.33×10⁹ CFU/L of *Thalassospira mesophila* strain BP3.

In a more preferred embodiment, the composition of the present disclosure comprises a mixture of *Halopseudomonas sp. strain BP4, Alloalcanivorax sp. strain BP14* and *Thalassospira mesophila* strain BP3; preferably in a proportion 1:1:1 in CFU.

In an embodiment, the composition of the present disclosure comprises a mixture of 10⁶ - 10¹² CFU/L of *Halopseudomonas sp. strain BP4,* 10⁶ - 10¹² CFU/L of *Alloalcanivorax sp. strain BP14* and 10⁶ - 10¹² CFU/L of *Thalassospira mesophila* strain BP3.

In an embodiment, the composition of the present disclosure comprises a mixture of 0.33×10⁹ CFU/L of *Halopseudomonas sp. strain BP4,* 0.33×10⁹ CFU/L of *Alloalcanivorax sp. strain BP14* and 0.33×10⁹ CFU/L of *Thalassospira mesophila* strain BP3.

In a preferred embodiment, the composition of the present disclosure comprises a synergistically effective amount of at least a bacterial strain defined in any of the previous claims.

Another aspect of the present disclosure relates to the use of the composition as a petroleum hydrocarbons pollutants remover or degrader; preferably as a seawater petroleum hydrocarbons pollutants remover or degrader.

Another aspect of the present disclosure relates to the use of the composition as an enhancing composition for the decomposition of petroleum hydrocarbons pollutants in sea water. In a preferred embodiment, the petroleum hydrocarbons pollutants are selected from turbine oil, crude oil and diesel oil, or mixtures thereof.

In a preferred embodiment, the amount of composition used per each litre of sea water is at least 10⁶ CFU (Colony Forming Unit); preferably ranges from 10⁶-10¹² CFU; more preferably 10⁹ CFU.

Another aspect of the present disclosure relates to a method for *in situ* bioremediation of a petroleum hydrocarbons pollutant contamination in sea or sea water sample comprising the following steps:
add to the sea or sea water sample a first dose of the composition described in any of the previous claims, wherein the amount of composition used per each litre of water is at least 10⁶ CFU; preferably ranges from 10⁶-10¹² CFU; more preferably 10⁹ CFU;
optionally add to the sea or sea water sample a second dose of the composition described in any of the previous claims, wherein the amount of composition used per each litre of water is at least 10⁶ CFU; preferably ranges from 10⁶-10¹² CFU; more preferably 10⁹ CFU.

### BRIEF DESCRIPTION OF THE DRAWINGS

The following figures provide preferred embodiments for illustrating the disclosure and should not be seen as limiting the scope of invention.

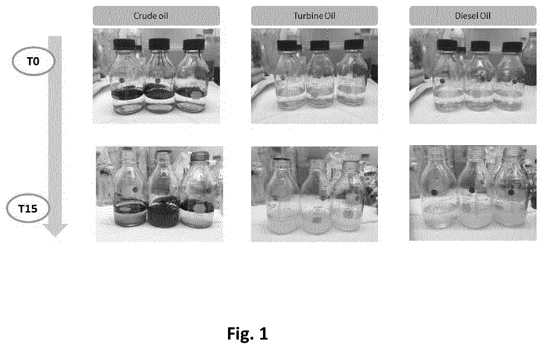

**Figure 1****:** Visual inspection of the flasks along the time of the enrichment experiment from where the isolates were originally collected, revealed a clear blending between the oils and the seawater after 15 days incubation.
**Figure 2A****:** Ven diagram representing the common genera between the three oils enrichments (CO - crude oil; TO - turbine oil; DO - diesel oil).
**Figure 2B****:** Histogram representing the total number of genera between the three oils enrichments (CO - crude oil; TO - turbine oil; DO - diesel oil).
**Figure 3****:** Abundance of hydrocarbon degraders, evaluated by the most-probable number (MPN) method, for the bacterial strains BP2, BP3, BDO24, BP4, BP14 and for the mix consortium.

### DETAILED DESCRIPTION

The present disclosure relates to a synergistic bioremediation composition for hydrocarbon pollutants degradation, comprising at least a bacterial strain of the genus *Pseudomonas* or *Halopseudomonas,* preferably *Pseudomonas segetis* strain BDO24 or *Halopseudomonas sp.* strain BP4; at least a bacterial strain of the genus *Alcanivorax* or *Alloalcanivorax,* preferably *Alcanivorax borkumensis* strain BP2 or *Alloalcanivorax sp.* strain BP14; and at least a bacterial strain of genus *Thalassospira,* preferably *Thalassospira mesophila* strain BP3.

This bioremediation product can be applied as a complete treatment in case of small or chronic spills, or as complement to the traditional remediation methods (physical and chemical) in case or larger spills.

The composition of the present disclosure provides several advantages: it promotes the use of autochthonous microorganisms, an environmental-friendly approach. It represents a big advantage because autochthonous bacteria are better adapted to the affected environment, when compared to exogenous bacteria, and proved to have better efficiency in pollutant degradation, so they can be also more successful in cleaning the oil spills. Besides this, the present invention solves the safety problem of using exogenous microorganisms that can cause unpredictable/negative ecological impacts into a particular marine environment.

The present disclosure represents a new resource for the maritime transport sector and port authorities to tackle oil spills, based on environmentally friendly solutions as the bioremediation solutions based on autochthonous microorganisms. The present invention is specially designed to be used for regions of the Atlantic Iberian Coast exposed to potential oil spills.

The following table provides information regarding the growth conditions and long-term preservation conditions for *Alcanivorax borkumensis* strain BP2, *Thalassospira mesophila* strain BP3, *Halopseudomonas sp.* strain BP4, *Alloalcanivorax sp.* strain BP14 and *Pseudomonas segetis* strain BDO24.

| **Strain** | **Accession number** | **Growth conditions** | | **Long-term preservation conditions** | |
|---|---|---|---|---|---|
| *Alcanivorax borkumensis* strain BP2 | CECT 30863 | MA (Marine Agar) Solid Medium: | | Cryopreservation in cryogenic tubes (2 ml) prepared as follows: | |
| | | | 55.20 g of marine agar | | 2 loops of biomass sample |
| | | | Deionized water (enough to reach 1L of volume) | | 1 ml of saline solution (NaCl concentration 0.85 % (w/v)) |
| | | | 28 °C, growth in the absence of light No aeration is used). | | 350 µL of glycerol (concentration 80 % (v/v)) |
| *Thalassospira mesophila* strain BP3 | CECT 30864 | MA (Marine Agar) Solid Medium: | | Cryopreservation in cryogenic tubes (2 ml) prepared as follows: | |
| | | | 55.20 g of marine agar | | 2 loops of biomass sample |
| | | | Deionized water (enough to reach 1L of volume) | | 1 ml of saline solution (NaCl concentration 0.85% (w/v)) |
| | | | 28 °C, growth in the absence of light No aeration is used. | | 350 µL of glycerol (concentration 80% (v/v)) |
| *Halopseudomonas sp*. strain BP4 | CECT 31085 | MA (Marine Agar) Solid Medium: | | Cryopreservation in cryogenic tubes (2 mL) prepared as follows: | |
| | | | 55.20 g of Marine Agar (MA) (CondaLab, Spain) | | |
| | | | | | 2 loops of biomass sample |
| | | | Deionized water (enough to reach 1L of volume) | | 1 mL of saline solution (NaCl concentration 0.85 °/) |
| | | | 28 °C, growth in a period of 3-4 days, in the absence of light | | |
| | | | | | 350 µL of glycerol (concentration 80 %) |
| | | | No aeration is used. | | |
| *Alloalcanivorax sp.* strain BP14 | CECT 31086 | MA (Marine Agar) Solid Medium: | | Cryopreservation in cryogenic tubes (2 mL) prepared as follows: | |
| | | | 55.20 g of Marine Agar (MA) (CondaLab, Spain) | | |
| | | | Deionized water (enough to reach 1L of volume) | | 2 loops of biomass sample |
| | | | | | 1 mL of saline solution (NaCl concentration 0.85 °/) |
| | | | 28 °C, growth in a period of 3-4 days, in the absence of light No aeration is used. | | 350 µL of glycerol (concentration 80 %) |
| *Pseudomonas segetis* strain BDO24 | CECT 30862 | **M1 Solid Medium:** | | Cryopreservation in cryogenic tubes (2 ml) prepared as follows: | |
| | | | 10 g of soluble starch | | |
| | | | 4 g of yeast extract | | |
| | | | 2 g of meat-derived peptone | | |
| | | | 15 g of plate count Agar | 2 loops of biomass sample | |
| | | | Filtered marine water (enough to reach 1L of volume) | 1 ml of saline solution (NaCl concentration 0.85% (w/v)) | |
| | | | | 350 µL of glycerol (concentration 80% (v/v)) | |
| | | | 28 °C, growth in the absence of light | | |
| | | | No aeration is used. | | |

### EXAMPLE 1

Example 1 of the present disclosure concerns to the composition according to **Table 1.**

**Table 1. Composition of example 1 of the present disclosure**

| **Component** | **Quantity (per litre)** |
|---|---|
| *Pseudomonas segetis* strain BDO24 | 0.33x10⁹ CFU |
| *Alcanivorax borkumensis* strain BP2 | 0.33x10⁹ CFU |
| *Thalassospira mesophila* strain BP3 | 0.33x10⁹ CFU |
| KNO₃ | 40 mM |
| KH₂PO₄ | 8 mM |

### RESULTS

**Table 2** resumes the results on removal percentage of total petroleum hydrocarbons (TPHs) by each strain when used isolated and by the composition of example 1 of the present disclosure.

**Table 2. Removal percentage of total petroleum hydrocarbons (TPHs)**

| **Strain/composition** | **Quantity per each litre of sea water** | **Result*** |
|---|---|---|
| *Pseudomonas segetis* strain BDO24 | 10⁹ CFU | 20%-40% |
| *Alcanivorax borkumensis* strain BP2 | 10⁹ CFU | 20%-40% |
| *Thalassospira mesophila* strain BP3 | 10⁹ CFU | 20%-40% |
| Composition of example 1 | 10⁹ CFU | 50%-90% |

| | | |
|---|---|---|
| *The result was quantified by Fourier transform infrared spectrophotometry comparing the total petroleum hydrocarbons in the sample at the beginning (t=0 days) and at the end (t= 15 days). The experiments were conducted in 50 mL sterilized glass flaks, in triplicate for each oil (crude oil, turbine oil and diesel oil). Each flask contained 10 mL of sterile seawater (for the strains isolated) or natural seawater (for the mixture/composition) sample supplied with the nutrients KNO₃ (40 mM) and KH₂PO₄ (8 mM), which served as exogenous nitrogen and phosphorous sources. The only carbon sources provided were: crude oil, diesel oil and turbine oil, supplied by an oil refinery. This was added to the flasks in a proportion of 20:0.5 (v/v), after passing through a 0.2 µm sterile filter to remove any microorganisms that could contaminate our samples. The carbon from the oils together with the nutrients supplied formed a ratio of C/N/P of 100:10:1. The flasks were incubated for 15 days under constant orbital agitation (100 rpm), at room temperature, in aerobic and aphotic conditions to avoid photo oxidation of the petroleum compounds. | | |

As depicted in the table above, it was observed that a bioremediation composition comprising *Pseudomonas segetis, Alcanivorax borkumensis* and *Thalassospira mesophila* provides a synergistic effect by providing an enhanced degradation activity of diesel oil, crude oil and/or turbine oil, comparing with the isolated use of each strain.

### EXAMPLE 2

**Table 3. Composition of example 2 of the present disclosure**

| **Component** | **Quantity (per litre)** |
|---|---|
| *Halopseudomonas* sp. strain BP4 | 0.33x10⁹ CFU |
| *Alloalcanivorax sp.* strain BP14 | 0.33x10⁹ CFU |
| *Thalassospira mesophila* strain BP3 | 0.33x10⁹ CFU |
| KNO₃ | 40 mM |
| KH₂PO₄ | 8 mM |

### Results

**Table 4** resumes the results on removal percentage of total petroleum hydrocarbons (TPHs) by each strain when used isolated and by the composition of example 2 of the present disclosure.

**Table 4. Removal percentage of total petroleum hydrocarbons (TPHs)**

| **Strain/composition** | **Quantity per each litre of sea water** | **Result *** |
|---|---|---|
| *Halopseudomonas* sp. strain BP4 | 10⁹ CFU | 30%-60% |
| *Alloalcanivorax dieselolei* strain BP14 | 10⁹ CFU | 20%-60% |
| *Thalassospira mesophila* strain BP3 | 10⁹ CFU | 20%-40% |
| Composition of example 2 | 10⁹ CFU | 50%-90% |

| | | |
|---|---|---|
| *The result was quantified by Fourier transform infrared spectrophotometry comparing the total petroleum hydrocarbons in the sample at the beginning (t=0 days) and at the end (t= 15 days). The experiments were conducted in 50 mL sterilized glass flaks, in triplicate for each oil (crude oil, turbine oil and diesel oil). Each flask contained 10 mL of sterile seawater (for the strains isolated) or natural seawater (for the mixture/composition) sample supplied with the nutrients KNO₃ (40 mM) and KH₂PO₄ (8 mM), which served as exogenous nitrogen and phosphorous sources. The only carbon sources provided were: crude oil, diesel oil and turbine oil, supplied by an oil refinery. This was added to the flasks in a proportion of 20:0.5 (v/v), after passing through a 0.2 µm sterile filter to remove any microorganisms that could contaminate our samples. The carbon from the oils together with the nutrients supplied formed a ratio of C/N/P of 100:10:1. The flasks were incubated for 15 days under constant orbital agitation (100 rpm), at room temperature, in aerobic and aphotic conditions to avoid photo oxidation of the petroleum compounds. | | |

As depicted in the table above, it was observed that a bioremediation composition comprising *Halopseudomonas* sp., *Alloalcanivorax* sp. and *Thalassospira mesophila* provides a synergistic effect by providing an enhanced degradation activity of diesel oil, crude oil and/or turbine oil, comparing with the isolated use of each strain.

### Materials and methods

Five bacterial strains, *Pseudomonas segetis* strain BDO24, *Alcanivorax borkumensis* strain BP2, *Thalassospira mesophila* strain BP3, *Halopseudomonas* sp. strain BP4 and *Alloalcanivorax* sp. strain BP14 previously isolated from seawater collected in Port of Leixões (Matosinhos, Portugal), were used in the current study due to their individual potential to degrade petroleum hydrocarbons.

Seawater sampling: for this study, seawater samples were collected from the Port of Leixões (Matosinhos, Portugal) in March 2020. A total of 10 L of seawater were collected at the surface with a sterile container, and stored in a refrigerated box, till further processing at CIIMAR laboratories. The surface water was characterized in terms of pH, salinity, temperature, conductivity, and oxygen levels by using a multiparameter probe (YSI EXO1 Sonde). Seawater samples were then filtered through Sterivex^{™} filters and processed in triplicate for oils enrichment experiments and estimation of the MPN (most probable number) of hydrocarbon-degrading microorganisms. All material was chemically decontaminated with 10 % (v/v) HCl solution and, afterward, washed with deionized water (conductivity <0.2 µS cm-1). The materials for microbial enrichments were sterilized by autoclaving at 121°C for 20 min and plastic materials were sterilized by UV light for 30 min.

Enrichment experiment: After seawater collection, enrichment experiments were conducted in 500 mL sterilized glass flaks, in triplicate for each site. Each flask contained 150 mL of the seawater sample supplied with the nutrients KNO₃ (40 mM) and KH₂PO₄ (8 mM), which served as exogenous nitrogen and phosphorous sources. The only carbon sources provided were: Arabian light crude-oil, diesel oil and turbine oil, all supplied by an oil refinery. This was added to the flasks in a proportion of 20:0.5 (v/v), after passing through a 0.2 µm sterile filter to remove any microorganisms that could contaminate our samples. The carbon from the oils together with the nutrients supplied formed a ratio of C/N/P of 100:10:1. The flasks were incubated for 2 weeks under constant orbital agitation (100 rpm), at room temperature, in aerobic and aphotic conditions to avoid photo oxidation of the petroleum compounds. After 15 days of incubation, 1 mL of medium from each triplicate flask was collected with sterile syringes to determine the abundance of hydrocarbon degraders and afterward all three replicates were combined for DNA isolation.

Samples collected from the sampling sites were immediately filtered, in duplicate, through Sterivex^{™} filters with a pore size of 0.22 µm, hydrophilic, PVDF, Durapore membrane (SVGV1010RS, Merck Millipore, Portugal) with the help of 50 mL sterile syringes, a PowerVac^{™} Manifold (Qiagen) and a vacuum pump, following the methodologies used in the Ocean Sampling Day event (Kopf et al., 2015). The volume filtered for each replicate was approximately 2L of seawater or the maximum volume possible in a 2-h window (ranging from 1.8 to 2L). Then, the remaining liquid in the filters was removed by using a syringe and the Sterivex^{™} was capped with the Inlet and Outlet caps, placed in sterile bags properly identified and stored in a -80°C freezer. For the samples from the enrichments with oils, the composite sample of the three replicates was also filtered using the same process described for natural samples. Due to the thick consistency of the three oils, the maximum volume possible was filtered (around 30 mL), in a 2-h time frame or until the Sterivex^{™} units clogged. Afterward, the remaining liquid in the filters was removed by using a syringe and the Sterivex^{™} was capped with the Inlet and Outlet caps, placed in sterile bags properly identified and stored in a -80°C freezer.

### DNA library preparation and amplicon sequencing

Sterivex^{™} filters resultant from filtration of natural and enriched communities were used for DNA extraction. DNA was extracted with the DNeasy^{®} PowerWater^{®} Sterivex Kit (QIAGEN, Inc.) and quantified fluorometrically using the Qubit dsDNA HS Assay (Thermo Fisher Scientific, Waltham, MA, United States). The amplification of the V4-V5 region of the genetic marker 16S rRNA gene was performed by using the primer pair 515YF (5'-GTGYCAGCMGCCGCGGTAA-3') (**Seq. ID. 1**) and Y926R-jed (5'-CCGYCAATTYMTTTRAGTTT-3') (**Seq. ID. 2**), according to Earth Microbiome Project protocols (Gilbert et al., 2014). These primers were adapted from the primer pair 515F/806R, that only amplified the V4 region, designed by Caporaso et al. (2011, 2012), and latter modified by Apprill et al. (2015) and Parada et al. (2016).

Sequencing of 16S rRNA gene amplicons was performed in Biocant - Biotechnology Park (Cantanhede, Portugal). Two Polymerase Chain Reaction (PCR) rounds were performed to amplify first the DNA with the specific primers and reamplify afterward to add sequencing adapters and dual indexes. The KAPA HiFi HotStart PCR kit was used to perform the PCR reactions, which included 0.3 µM of each primer and 12.5 ng of template DNA in a total volume of 25 µL. Briefly, the PCR protocol included a 3 min denaturation step, followed by 25 cycles of 98°C for 20 s, 60°C for 30 s, and 72°C for 30 s, and, finally, an extension stage at 72°C for 5 min. A second PCR reaction was performed to add indexes and sequencing adapters to the target region, according to manufacturer's recommendations (Illumina, 2013). Negative controls without template were included in all PCR reactions. Lastly, PCR products were one-step purified and normalized using SequalPrep 96-well plate kit (Thermo Fisher Scientific, Waltham, MA, United States), pooled, and pair-end sequenced in the Illumina MiSeq^{®} sequencer using 2 × 300 bp with the V3 chemistry, according to manufacturer instructions (Illumina, San Diego, CA, United States) at Genoinseq (Cantanhede, Portugal).

Bioinformatic analysis: Raw reads provided by the Genoinseq sequencing company were then trimmed for primer removal using "cutadapt" v.1.16 and imported into R (version 3.5.1) using "DADA2" package v.1.10.1 (Callahan et al., 2016a). Dada2 algorithm with default settings was used to sample filtering, trimming, error rates learning, dereplication, chimera removing and amplicon sequence variant (ASV) inference (Callahan, 2016) providing as output an ASV abundance table.

Taxonomy assignation of the ASV representative sequences was carried out against SILVA v 132 database (Quast, 2012) trained with a naïve Bayes classifier against the same the V4-V5 region of 16S rRNA gene target for sequencing. ASV assigned to non-target sequences (e.g "Eukarya", Choloroplasts", "Mitochondria") and not classified at Kingdom level were removed and excluded from the analysis. Taxonomy plots were created using the ggplot2' package in R environment (version 3.2.2. Copyright 2015 The R Foundation for Statistical Computing).

Statistical Analysis and Data Visualization: Mean values (n = 3) and respective standard deviations of MPN and TPHs concentrations were determined per treatment. Significant relationships were considered when p values were below 0.05 (wilcoxon test p value). BarPlots were obtained in the R environment (version 3.2.2. Copyright 2015 The R Foundation for Statistical Computing), using base R and the "ggplot2" package.

Hydrocarbon Degraders Abundance: For this work, the Most Probable Number (MPN) procedure was adapted from Wrenn and Venosa (1996) to determine the abundance of hydrocarbon-degrading bacteria in natural samples and enriched samples. This method was performed, in triplicate, in 96-well microtiter plates using the growth medium Bushnell Haas medium supplemented with 2% NaCl. To determine the total HC degraders, the selective substrate was pre-filtered (0.2 µm) Arabian light crude-oil, turbine oil and diesel oil, according to the description available in Gouveia et al. (2018). The abundance of HC degrading microorganisms was then estimated using the Most Probable Number Calculator v3.1 program (Klee, 1993). The MPN method was applied to the selected isolated strains and to the mixed consortium.

Bacteria isolation and phylogenetic identification: After the enrichment phase, bacteria present in the microbial culture were spread in 3 media culture Marine Agar (MA), M1 and Bushnell- Haas (BH). Different colonies were described morphologically and purified. Isolated strains were then cryopreserved at -80 °C and biomass of each strain was also collected for phylogenetic identification. Bacterial DNA was extracted from all isolates with the kit E.Z.N.A. ^{®} Bacterial DNA (Omega Bio-tek). DNA quantification was performed with the kit Quant-it HsDNA in the Qubit fluorometer (Invitrogen). For phylogenetic identification, the full-length of the bacterial 16S rRNA gene was amplified with the universal primers 27F (5'-AGAGTTTGATCMTGGCTCAG-3') (**Seq. ID. 3**) and 1492R (5'-TACGGYTACCTTGTTACGACTT-3') (**Seq. ID. 4**) by Polymerase Chain Reaction (PCR).

The PCR mixture and PCR conditions were the same as Perdigão et al., 2021. Amplified samples were run in a 1.5% agarose gel containing SYBR Safe (Thermo Fisher Scientific, Waltham, MA, USA) and the resulting PCR products were sequenced at Genomics i3S Scientific Platform (Porto, Portugal). 16S rRNA sequences obtained were analyzed using the Geneious software (11.1.4) and the consensus sequences were identified using the NCBI BLAST database (https://blast.ncbi.nlm.nih.gov/Blast.cgi, accessed on 20 June 2024). The 16S rRNA gene sequences of the identified bacterial strains were deposited in GenBank (NCBI, Bethesda, MD, USA).

**Table 5. The following table describes the list of sequences described in the present application.**

| **Seq. ID** | **Sequence name** | **Organism name** | **Molecule type** | **Qualifier Molecule Type** | **Residues** |
|---|---|---|---|---|---|
| **Seq. ID. 1** | Primer pair 515YF | synthetic construct | DNA | other DNA | GTGYCAGCMGCCGCGGTAA |
| **Seq. ID. 2** | Primer pair Y926R-jed | synthetic construct | DNA | other DNA | CCGYCAATTYMTTTRAGTTT |
| **Seq. ID 3** | Primer pair 27F | synthetic construct | DNA | other DNA | AGAGTTTGATCMTGGCTCAG |
| **Seq. ID 4** | Primer pair 1492R | synthetic construct | DNA | other DNA | TACGGYTACCTTGTTACGACTT |

### Results

### 16S rDNA metabarcoding analysis

Sequencing analysis yielded a total of 667323 raw sequences, which after the DADA2 pipeline, decreased to 440098. The number of raw reads recovered from the natural community DNA samples varied between 86234 and 91661 and the number of raw reads recovered from samples after the oils enrichments varied between 65047 and 110338. After bioinformatics analysis, the total number of sequences present in natural communities ranged between 53554 and 57820 and the total number of sequences in enriched communities ranged between 41886 and 75456.

### Microbial communities Taxonomic Profiles in Natural and Oils Enrichment Samples

Relevant Phyla Natural prokaryotic community characterization at the highest taxonomic level showed that these communities presented high relative abundances for the phyla Proteobacteria (≈ 30-38%), Bacteroidota (≈ 32%), Actinobacteria (≈ 9%), Cyanobacteria (≈ 0.02-13%), Planctomycetota (≈ 11%), and Firmicutes (≈ 7%). In the enriched samples, the selected prokaryotic communities were composed predominately by Proteobacteria (approximately between 48 and 70%) and Bacteroidota (≈ 30-52%). Moreover, natural microbial communities showed high relative abundance of not assigned sequences at the phylum level, in comparison to oil-enriched communities.

### Taxonomic Identification of the Isolated Strains

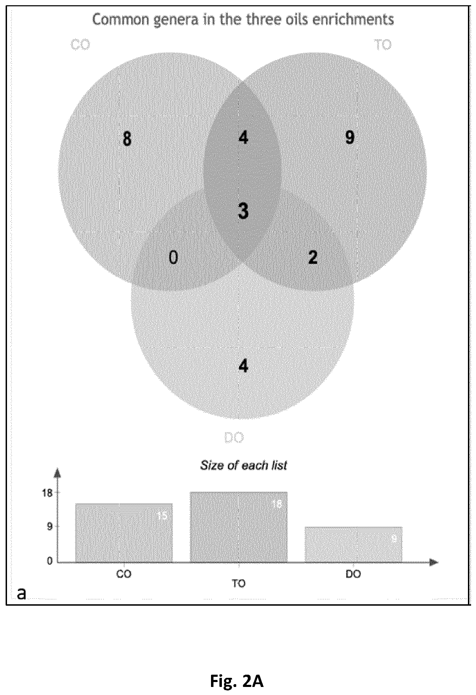

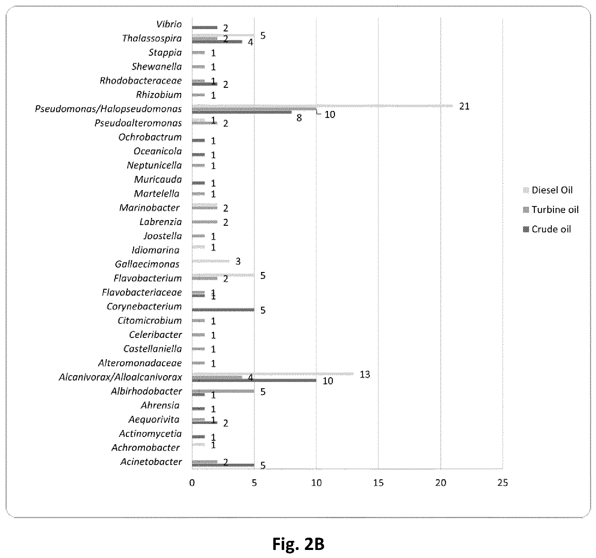

From the Leixões the enrichment experiment with crude oil turbine oil and diesel oil, 145 bacterial strains were isolated and phylogenetically identified. Common genera and total number of genera between the three oils are represented with a Ven Diagram (http://jvenn.toulouse.inra.fr/app/index.html) in Figure 2a, and with a histogram in figure 2b.

The Ven Diagram (**Figure 2A**) shows in an intersection between three circles, corresponding to the three oils, the common genera between them: *Alcanivorax*/*Alloalcanivorax, Pseudomonas*/*Halopseudomonas* and *Thalassospira.* The genera in common between crude oil and turbine oil are *Acinetobacter, Albirhodobacter* and the families Flavobacteriaceae and Rhodobacteriaceae. The genera in common between turbine oil and diesel oil are: *Flavobacterium* and *Marinobacter.* The **Figure 2b** represents the total number of bacterial genera present in each oil enrichment. The most numerous ones in three enriched oils are *Pseudomonas*/*Halopseudomonas* (21 isolates in diesel, 10 in turbine oil and 8 in crude oil) and *Alcanivorax*/*Alloalcanivorax* (13 isolates in diesel, 4 in turbine oil and 10 in crude oil). For the other bacterial genera, 5 or less bacterial strains were recovered in each oil.

### Hydrocarbons-Degrading Bacterial Consortium

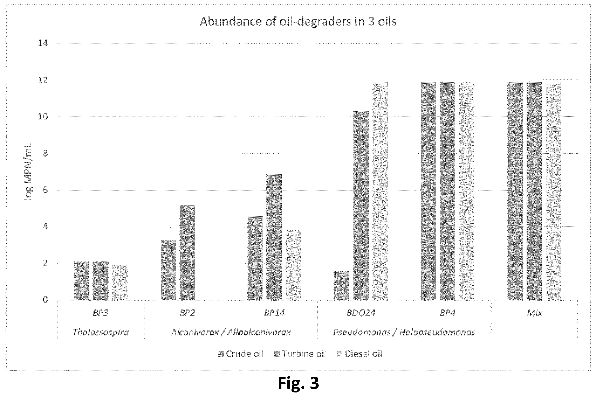

The hydrocarbon degrading potential of the 5 bacterial strains (BP4, BP14, BP3, BP2, BDO24), selected as the most promise ones among the 145 isolates obtained from the enrichment process with crude oil, diesel oil or turbine oil, was tested individually and as a developed composition comprising the mixed consortium (MIX). Results from MPN showed that all the individual strains and the mixture of the three strains (MIX), displayed an ability to degrade petroleum hydrocarbons (**Figure 3**). Surprisingly, higher abundances of hydrocarbon-degraders and a synergistic effect was observed for the mix consortium, which showed densities >10¹¹ MPN/mL for all the tested oils (crude oil, diesel oil or turbine oil.

In this work, a bioremediation consortium based on three strains of hydrocarbon-degrading bacteria isolated from Port of Leixões (Matosinhos, Portugal), was developed. The development of the bioremediation consortium was based on the use of both culture-dependent and independent approaches, for the characterization and the isolation of the key components of the hydrocarbon degrading community. A total of 145 bacterial strains were isolated and identified after enrichment with the three hydrocarbon contaminants (crude oil, turbine oil and diesel oil) by full-length 16S rRNA gene Sanger sequencing. In parallel, environmental prokaryotic communities were characterized, before and after the enrichment, through V4-V5 16S rRNA gene amplicon sequencing (Illumina MiSeq). These indicator taxa are mainly dominated by genera known to degrade hydrocarbons, namely *Alcanivorax*/*Alloalcanivorax, Thalassospira,* and *Pseudomonas*/*Halopseudomonas* which were also possible to cultivate in the lab. The hydrocarbon degrading potential of the 5 bacterial strains (*Pseudomonas segetis* strain BDO24, *Alcanivorax borkumensis* strain BP2, *Thalassospira mesophila* strain BP3, *Halopseudomonas* sp. strain BP4, *Alloalcanivorax dieselolei* strain BP14), selected as the most promise ones, was tested individually and as a novel mixed consortium (MIX), developed and tested for the first time. The consortium of three of the bacterial strains proved to act synergistically in enhancing the degradation of crude oil, turbine oil and diesel oil, when applied as an autochthonous bioaugmentation inoculum to microcosm experiments with natural seawater and nutrients.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The following dependent claims further set out particular embodiments of the disclosure.

### References

1. Crisafi, F.; Genovese, M.; Smedile, F.; Russo, D.; Catalfamo, M.; Yakimov, M.; Giuliano, L., and Denaro, R. Bioremediation technologies for polluted seawater sampled after an oil-spill in Taranto Gulf (Italy): A comparison of biostimulation, bioaugmentation and use of a washing agent in microcosm studies. Marine pollution bulletin 2016. 106(1-2), 119-126. DOI: 10.1016/j.marpolbul.2016.03.017.
2. Barron, M.G. Ecological Impacts of the Deepwater Horizon Oil Spill: Implications for Immunotoxicity. Toxicologic Pathology 2012. 40(2), 315-320. DOI: 10.1177/0192623311428474.
3. Atlas, R.M. and Hazen, T.C. Oil biodegradation and bioremediation: a tale of the two worst spills in U.S. history. Environ Sci Technol 2011. 45(16), 6709-15. DOI: 10.1021/es2013227.
4. Almeida, C.M.; Reis, I.; Couto, M.N.; Bordalo, A.A., and Mucha, A.P. Potential of the microbial community present in an unimpacted beach sediment to remediate petroleum hydrocarbons. Environ Sci Pollut Res Int 2013. 20(5), 3176-84. DOI: 10.1007/s 11356-012-1240-2.
5. Li, X.; Zhao, L., and Adam, M. Biodegradation of marine crude oil pollution using a salt-tolerant bacterial consortium isolated from Bohai Bay, China. Mar Pollut Bull 2016. 105(1), 43-50. DOI: 10.1016/j.marpolbul.2016.02.073.
6. Priya, A.; Manab Sarma, P., and Lal, B. Isolation and characterization ofCandida vishwanathiistrain TERI MS1 for degradation of petroleum hydrocarbons in marine environment. Desalination and Water Treatment 2016. 57(46), 22099-22106. DOI: 10.1080/19443994.2015.1134351.
7. Nikolopoulou, M.; Pasadakis, N., and Kalogerakis, N. Evaluation of autochthonous bioaugmentation and biostimulation during microcosm-simulated oil spills. Mar Pollut Bull 2013. 72(1), 165-73. DOI: 10.1016/j.marpolbul.2013.04.007.
8. Perdigão, R.; Almeida, C.M.R.; Santos, F.; Carvalho, M.F., and Mucha, A.P. Optimization of an Autochthonous Bacterial Consortium Obtained from Beach Sediments for Bioremediation of Petroleum Hydrocarbons. Water 2021. 13(1), 66. DOI: 10.3390/w13010066.
9. Maamar, A.; Lucchesi, M.-E.; Debaets, S.; Nguyen van Long, N.; Quemener, M.; Coton, E.; Bouderbala, M.; Burgaud, G., and Matallah-Boutiba, A. Highlighting the Crude Oil Bioremediation Potential of Marine Fungi Isolated from the Port of Oran (Algeria). Diversity 2020. 12(5), 196. DOI: 10.3390/d12050196.
10. Zinjarde, S. and Pant, A. Hydrocarbon degraders from tropical marine environments. Marine pollution bulletin 2002. 44(2), 118-121. DOI: 10.1016/S0025-326X(01)00185-0.
11. Leahy, J.G. and Colwell, R.R. Microbial degradation of hydrocarbons in the environment. Microbiological reviews 1990. 54(3), 305-315. DOI: 0146-0749/90/030305-11$02.00/0.
12. Djahnit, N.; Chernai, S.; Catania, V.; Hamdi, B.; China, B.; Cappello, S., and Quatrini, P. Isolation, characterization and determination of biotechnological potential of oil-degrading bacteria from Algerian centre coast. Journal of applied microbiology 2019. 126(3), 780-795. DOI: doi.org/10.1111/jam.14185.
13. Harwati, T.U.; Kasai, Y.; Kodama, Y.; Susilaningsih, D., and Watanabe, K. Characterization of diverse hydrocarbon-degrading bacteria isolated from Indonesian seawater. Microbes and Environments 2007. 22(4), 412-415. DOI: 10.1264/jsme2.22.412.
14. Wang, W.; Zhong, R.; Shan, D., and Shao, Z. Indigenous oil-degrading bacteria in crude oil-contaminated seawater of the Yellow sea, China. Applied microbiology and biotechnology 2014. 98(16), 7253-7269.
15. Hosokawa, R.; Nagai, M.; Morikawa, M., and Okuyama, H. Autochthonous bioaugmentation and its possible application to oil spills. World Journal of Microbiology & Biotechnology 2009. 25(9), 1519-1528. DOI: 10.1007/s11274-009-0044-0.
16. Varjani, S.J. Microbial degradation of petroleum hydrocarbons. Bioresource technology 2017. 223, 277-286. DOI: 10.1016/j.biortech.2016.10.037.
17. Fuentes, S.; Mendez, V.; Aguila, P., and Seeger, M. Bioremediation of petroleum hydrocarbons: catabolic genes, microbial communities, and applications. Appl Microbiol Biotechnol 2014. 98(11), 4781-94. DOI: 10.1007/s00253-014-5684-9.
18. Souza, E.C.; Vessoni-Penna, T.C., and de Souza Oliveira, R.P. Biosurfactant-enhanced hydrocarbon bioremediation: An overview. International biodeterioration & biodegradation 2014. 89, 88-94. DOI: 10.1016/j.ibiod.2014.01.007.
19. Dehvari, M.; Ghafari, S.; Haghighifard, N.J., and Jorfi, S. Petroleum Contaminated Seawater Detoxification in Microcosm by Halotolerant Consortium Isolated from Persian Gulf. Current Microbiology 2020, 1-12. DOI: 10.1007%2Fs00284-020-02267-x.
20. Pereira, E.; Napp, A.P.; Allebrandt, S.; Barbosa, R.; Reuwsaat, J.; Lopes, W.; Kmetzsch, L.; Staats, C.C.; Schrank, A., and Dallegrave, A. Biodegradation of aliphatic and polycyclic aromatic hydrocarbons in seawater by autochthonous microorganisms. International Biodeterioration & Biodegradation 2019. 145, 104789. DOI: 10.1016/j.ibiod.2019.104789.
21. Fodelianakis, S.; Antoniou, E.; Mapelli, F.; Magagnini, M.; Nikolopoulou, M.; Marasco, R.; Barbato, M.; Tsiola, A.; Tsikopoulou, I.; Giaccaglia, L.; Mahjoubi, M.; Jaouani, A.; Amer, R.; Hussein, E.; Al-Horani, F.A.; Benzha, F.; Blaghen, M.; Malkawi, H.I.; Abdel-Fattah, Y.; Cherif, A.; Daffonchio, D., and Kalogerakis, N. Allochthonous bioaugmentation in ex situ treatment of crude oil-polluted sediments in the presence of an effective degrading indigenous microbiome. J Hazard Mater 2015. 287, 78-86. DOI: 10.1016/j.jhazmat.2015.01.038.
22. Tao, K.; Liu, X.; Chen, X.; Hu, X.; Cao, L., and Yuan, X. Biodegradation of crude oil by a defined co-culture of indigenous bacterial consortium and exogenous Bacillus subtilis. Bioresour Technol 2017. 224, 327-332. DOI: 10.1016/j.biortech.2016.10.073.
23. Das, N. and Chandran, P. Microbial degradation of petroleum hydrocarbon contaminants: an overview. Biotechnol Res Int 2011. 2011, 941810. DOI: 10.4061/2011/941810.
24. Ivshina, I.B.; Kuyukina, M.S.; Krivoruchko, A.V.; Elkin, A.A.; Makarov, S.O.; Cunningham, C.J.; Peshkur, T.A.; Atlas, R.M., and Philp, J.C. Oil spill problems and sustainable response strategies through new technologies. Environmental Science-Processes & Impacts 2015. 17(7), 1201-1219. DOI: 10.1039/c5em00070j.
25. Xu, X.; Liu, W.; Tian, S.; Wang, W.; Qi, Q.; Jiang, P.; Gao, X.; Li, F.; Li, H., and Yu, H. Petroleum Hydrocarbon-Degrading Bacteria for the Remediation of Oil Pollution Under Aerobic Conditions: A Perspective Analysis. Frontiers in Microbiology 2018. 9(2885). DOI: 10.3389/fmicb.2018.02885.
26. Partovinia, A. and Rasekh, B. Review of the immobilized microbial cell systems for bioremediation of petroleum hydrocarbons polluted environments. Critical Reviews in Environmental Science and Technology 2018. 48(1), 1-38. DOI: 10.1080/10643389.2018.1439652.
27. Dai, X.; Lv, J.; Yan, G.; Chen, C.; Guo, S., and Fu, P. Bioremediation of intertidal zones polluted by heavy oil spilling using immobilized laccase-bacteria consortium. Bioresource Technology 2020, 123305.
28. Wang, X.; Liu, Y.; Song, C.; Yuan, X.; Zhang, Q., and Miao, Y. Application Analysis of Immobilized Bioremediation Preparation in Oil Spill Contaminated Shore. in IOP Conference Series: Earth and Environmental Science. 2020. IOP Publishing.
29. Alessandrello, M.J.; Tomás, M.S.J.; Raimondo, E.E.; Vullo, D.L., and Ferrero, M.A. Petroleum oil removal by immobilized bacterial cells on polyurethane foam under different temperature conditions. Marine Pollution Bulletin 2017. 122(1-2), 156-160. DOI: 10.1016/j.marpolbul.2017.06.040.
30. Chen, C.-H.; Whang, L.-M.; Pan, C.-L.; Yang, C.-L., and Liu, P.-W.G. Immobilization of diesel-degrading consortia for bioremediation of diesel-contaminated groundwater and seawater. International Biodeterioration & Biodegradation 2017. 124, 62-72. DOI: 10.1016/j.ibiod.2017.07.001.
31. Hou, D.; Shen, X.; Luo, Q.; He, Y.; Wang, Q., and Liu, Q. Enhancement of the diesel oil degradation ability of a marine bacterial strain by immobilization on a novel compound carrier material. Marine pollution bulletin 2013. 67(1-2), 146-151. DOI: 10.1016/j.marpolbul.2012.11.021.
32. Tsutsumi, H.; Kono, M.; Takai, K.; Manabe, T.; Haraguchi, M.; Yamamoto, I., and Oppenheimer, C. Bioremediation on the shore after an oil spill from the Nakhodka in the Sea of Japan. III. Field tests of a bioremediation agent with microbiological cultures for the treatment of an oil spill. Marine pollution bulletin 2000. 40(4), 320-324. DOI: 10.1016/S0025-326X(99)00220-9.
33. Villela, H.D.M.; Peixoto, R.S.; Soriano, A.U., and Carmo, F.L. Microbial bioremediation of oil contaminated seawater: A survey of patent deposits and the characterization of the top genera applied. Science of the Total Environment 2019. 666, 743-758. DOI: 10.1016/j.scitotenv.2019.02.153.

Girard L, Lood C, De Mot R, van Noort V, Baudart J. Genomic diversity and metabolic potential of marine Pseudomonadaceae. Front Microbiol. 2023 Apr 6;14:1071039. doi: 10.3389/fmicb.2023.1071039.
Girard L., Lood C., Höfte M., Vandamme P., Rokni-Zadeh H., van Noort V., et al.. (2021). The ever-expanding pseudomonas genus: description of 43 new species and partition of the pseudomonas putida group. Microorganisms 9:1766. doi: 10.3390/microorganisms9081766.
Lalucat J., Gomila M., Mulet M., Zaruma A., García-Valdés E. (2022). Past, present and future of the boundaries of the pseudomonas genus: proposal of Stutzerimonas gen. Nov. Syst. Appl. Microbiol. 45:126289. doi: 10.1016/j.syapm.2021.126289.
Rai, A., Suresh, G., Ria, B., L, V., Pk, S., Ipsita, S., Sasikala, C., and Venkata Ramana, C. "Phylogenomic analysis of the genus Alcanivorax: proposal for division of this genus into the emended genus Alcanivorax and two novel genera Alloalcanivorax gen. nov. and Isoalcanivorax gen. nov." Int. J. Syst. Evol. Microbiol. (2023) 73(1):005672.

## Claims

1. Bioremediation composition for hydrocarbon pollutants degradation comprising a synergistic mixture of:
at least a bacterial strain of genus *Pseudomonas or Halopseudomonas;*
at least a bacterial strain of genus *Alcanivorax* or *Alloalcanivorax;* and
at least a bacterial strain of genus *Thalassospira;*
wherein the bacterial strain of genus *Pseudomonas or Halopseudomonas* is selected from a list consisting of: *Pseudomonas marincola, Pseudomonas oceani, Pseudomonas putida, Pseudomonas sabulinigri, Pseudomonas segetis, Pseudomonas sp.* or *Halopseudomonas* sp., or combinations thereof; preferably *Pseudomonas segetis* or *Halopseudomonas* sp.;
wherein the bacterial strain of genus *Alcanivorax* or *Alloalcanivorax* is selected from a list consisting of: *Alcanivorax borkumensis, Alcanivorax dieselolei, Alcanivorax marinus, Alcanivorax xenomutans* or *Alloalcanivorax* sp., or combinations thereof; preferably *Alcanivorax borkumensis* or *Alloalcanivorax* sp.;
wherein the bacterial strain of genus *Thalassospira* is selected from a list consisting of: *Thalassospira lucentensis, Thalassospira mesophila, Thalassospira sp.* or *Thalassospira xiamenensis,* or combinations thereof; preferably *Thalassospira mesophila.*

2. Composition according to the previous claim wherein:
*Pseudomonas marincola* strain is selected from a list consisting of: BP5, BP24, BP25, BP45, BTO19, BTO22;
*Halopseudomonas oceani* strain is selected from a list consisting of: BTO18, BTO20, BTO37;
*Pseudomonas putida* strain is selected from a list consisting of BDO8, BDO20, BDO31, BDO49;
*Halopseudomonas sabulinigri* strain is selected from a list consisting of BTO13, BDO37;
*Pseudomonas segetis* strain is selected from a list consisting of BTO21, BTO44, BDO17, BDO22, BDO38, BDO46, BP43, BDO24; preferably *Pseudomonas segetis* strain BDO24;
*Pseudomonas sp.* or Halopseudomonas sp. strain is selected from a list consisting of BP4, BP17, BDO6, BDO7, BDO11, BDO12, BDO26, BDO36, BDO40, BDO42, BDO43, BDO45, BDO52, BP35, BTO6, BTO17, preferably *Halopseudomonas* sp. strain BP4.

3. Composition according to the previous claim 1 wherein:
*Alcanivorax borkumensis* strain is selected from a list consisting of: BP2, BP7, BP37, BP41, BTO2, BTO28, BTO31, BTO32, BDO1, BDO5, BDO44; preferably *Alcanivorax borkumensis* strain BP2;
*Alloalcanivorax dieselolei* strain is selected from a list consisting of: BP18, BP33, BDO3, BDO14, BDO25, BDO28, BDO32, BDO33, BDO34, BDO41, BDO48;
*Alloalcanivorax marinus* strain *is strain* BDO29;
*Alcanivorax sp. or Alloalcanivorax* sp. strain is selected from a list consisting of: BP14, BP8,
BP40; preferably *Alloalcanivorax* sp. BP14;
*Alloalcanivorax xenomutans* strain is strain BP21.

4. Composition according to the previous claim 1 wherein:
*Thalassospira lucentensis* strain is selected from a list consisting of: BP39, BP15, BDO19;
*Thalassospira mesophila* strain is selected from a list consisting of: BP3, BP30; preferably *Thalassospira mesophila* strain BP3;
*Thalassospira sp.* strain is selected from a list consisting of: BDO15, BDO23, BTO3, BTO45;
*Thalassospira xiamenensis* strain is selected from a list consisting of: BDO13, BDO30.

5. Composition according to any of the previous claims comprising 10⁶ - 10¹² CFU/L of a bacterial strain of genus *Pseudomonas;* preferably *Pseudomonas segetis* strain BDO24.

6. Composition according to any of the previous claims comprising 10⁶ - 10¹² CFU/L of a bacterial strain of genus *Halopseudomonas;* preferably *Halopseudomonas sp.* strain BP4.

7. Composition according to any of the previous claims comprising 10⁶ - 10¹² CFU/L of a bacterial strain of genus *Alcanivorax;* preferably *Alcanivorax borkumensis* strain BP2.

8. Composition according to any of the previous claims comprising 10⁶ - 10¹² CFU/L of a bacterial strain of genus *Alloalcanivorax*; preferably *Alloalcanivorax* strain BP14.

9. Composition according to any of the previous claims comprising 10⁶ - 10¹² CFU/L of a bacterial strain of genus *Thalassospira;* preferably *Thalassospira mesophila* strain BP3.

10. Composition according to any of the previous claims comprising *Pseudomonas segetis* strain BDO24, *Alcanivorax borkumensis* strain BP2 and *Thalassospira mesophila* strain BP3; preferably in a proportion 1:1:1 CFU.

11. Composition according to the previous claim comprising 10⁶ - 10¹² CFU/L of *Pseudomonas segetis* strain BDO24, 10⁶ - 10¹² CFU/L of *Alcanivorax borkumensis* strain BP2 and 10⁶ - 10¹² CFU/L of *Thalassospira mesophila* strain BP3; preferably comprising 0.33×10⁹ CFU/L of *Pseudomonas segetis* strain BDO24, 0.33×10⁹ CFU/L of *Alcanivorax borkumensis* strain BP2 and 0.33×10⁹ CFU/L of *Thalassospira mesophila* strain BP3.

12. Composition according to any of the previous claims comprising *Halopseudomonas sp.* strain BP4, *Alloalcanivorax sp.* strain BP14 and *Thalassospira mesophila* strain BP3; preferably in a proportion 1:1:1 CFU.

13. Composition according to the previous claim comprising 10⁶ - 10¹² CFU/L of *Halopseudomonas sp.* strain BP4, 10⁶ - 10¹² CFU/L of *Alloalcanivorax sp.* strain BP14 and 10⁶ - 10¹² CFU/L of *Thalassospira mesophila* strain BP3; preferably comprising 0.33×10⁹ CFU/L of *Halopseudomonas sp.* strain BP4, 0.33×10⁹ CFU/L of *Alloalcanivorax sp.* strain BP14 and 0.33×10⁹ CFU/L of *Thalassospira mesophila* strain BP3.

14. Use of the composition according to any of the previous claims as a petroleum hydrocarbons pollutants remover or degrader; preferably as a sea water petroleum hydrocarbons pollutants remover or degrader; more preferably the petroleum hydrocarbons pollutants are selected from turbine oil, crude oil and diesel oil, or mixtures thereof.

15. Method for in situ remediation of a petroleum hydrocarbons pollutant contamination in sea or sea water sample comprising the following steps:
add to the sea or sea water sample a first dose of the composition described in any of the previous claims, wherein the amount of composition used per each litre of water is at least 10⁶ CFU; preferably ranges from 10⁶ - 10¹² CFU; more preferably 10⁹ CFU;
optionally add to the sea or sea water sample a second dose of the composition described in any of the previous claims, wherein the amount of composition used per each litre of water is at least 10⁶ CFU; preferably ranges from 10⁶ - 10¹² CFU; more preferably 10⁹ CFU.
